# EUROPEAN PATENT SPECIFICATION

(11) **EP 3 304 026 B1**
(45) Date of publication and mention of the grant of the patent: **03.04.2024**
(21) Application number: 16733242.8
(22) Date of filing: 07.06.2016
(51) Int. Cl.: G01N 33/38, G01M 11/08

(54) **INTEGRATED CAPSTAN AND APPARATUS FOR SCREEN TESTING AN OPTICAL FIBER**
INTEGRIERTER CAPSTAN UND VORRICHTUNG FÜR ZUGSPANNUNGSTEST EINER OPTISCHEN FASER
CABESTAN INTÉGRÉ ET APPAREIL DE TEST DE SÉLECTION D'UNE FIBRE OPTIQUE

(30) Priority: 08.06.2015 US 201562172430 P
(43) Date of publication of application: 11.04.2018
(73) Proprietor: Corning Incorporated, Corning, New York 14831 (US)
(72) Inventor: LIU, Ziwei, Ft. Worth, Texas 76137 (US)
(74) Representative: Elkington and Fife LLP
(86) International application number: PCT/US2016/036175
(87) International publication number: WO 2016/200784

(56) References cited:
- EP-A1- 0 785 173
- EP-A2- 0 514 858
- JP-A- 2005 097 051
- US-A- 4 997 276
- US-A- 5 178 313
- US-A1- 2013 255 397

## Description

### Field

The present specification generally relates to screen testing optical fibers. More particularly, the present specification relates to an integrated capstan and systems for screening optical fibers incorporation the integrated capstan.

### Technical Background

Optical fibers are formed from preforms in a drawing process. The preform is a consolidated glass that includes a central core region circumscribed by one or more annular cladding regions. The core region is a higher index glass region (e.g. Ge-doped silica glass) and the one or more cladding regions are lower index glass regions (e.g. undoped silica glass or downdoped silica glass). To form the fiber, the preform is heated to a draw temperature sufficient to soften the glass and a glass fiber is pulled from the preform and driven through a processing apparatus by tension. The glass fiber includes a core and cladding and typically has a diameter of 125 µm.

The fiber manufacturing process further includes coating the glass fiber. The coating can be completed in a continuous online manner during draw or as a separate offline process independent of drawing the glass fiber. The coating process typically includes forming a soft primary coating on the glass fiber and a hard secondary coating on the primary coating. The secondary coating protects the glass fiber from external forces and the primary coating acts to dissipate stresses incident upon the secondary coating to prevent undue levels of stress from concentrating at the surface of the glass fiber. The fiber coating process includes applying liquid coating formulations onto the glass fiber and curing the formulations to produce solid coatings.

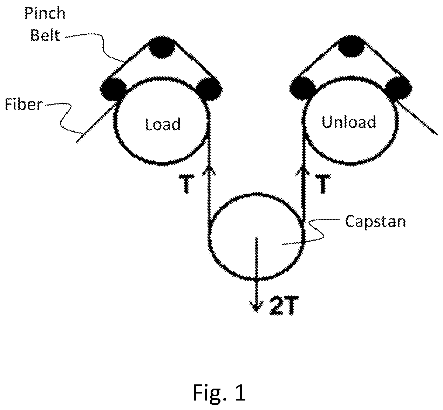

Before shipment to customers, coated fibers are tested for mechanical durability. The testing process is referred to as screen testing or proof testing and is illustrated in FIG. 1. In screen testing, a coated fiber is placed between capstan assemblies and subjected to a tensile stress T. The capstan assemblies consist of a capstan and pinch belt. The pinch belt assemblies consist of a capstan and a pinch belt. The pinch belts are used to apply a compressive load on the coated fiber. The compressive load localizes the tension used for screen testing in the section of coated fiber between the two belted capstan assemblies and isolates the section of coated fiber from the payout and winding tensions used in the rotation of capstans during conveyance of the coated fiber through the process apparatus.

As each pinch belt is pressed against its corresponding capstan, it imparts additional stresses to the coatings. The additional stresses may lead to failure or the formation of defects in the coatings. Damage to coatings is of particular concern for spliced fibers. When splicing two optical fibers, the primary and secondary coatings are stripped off the fiber ends and the bare glass ends are spliced. The spliced bare glass is then recoated with a single layer of coating (referred to as a "recoat"). The interface between the constituent fiber and the recoat is referred to as a splice joint. In order to prevent adhesion failures at the splice joint, a recoat material with a high modulus is required. When spliced fibers are screened through the belted capstan assemblies, the original and recoat sections of the fiber respond differently under the compressive load of the pinch belts because the original fiber section includes a low modulus primary coating, while the recoated section of fiber does not. When subjected to a compressive load, the original section of the fiber exhibits much higher compliance than the recoat section. The compliance mismatch results in additional strains in the secondary coating of the original section of fiber. Additionally, there is a shear stress mismatch between the original and recoat section of the fiber. The mismatch in shear stress results in large shear stresses on the coatings at the splice joint. The additional strains that the capstan assembly imparts on the fiber often results in a cohesive failure of the secondary coating of the original section of the fiber near the splice joint. There is thus a need to reduce the stresses in the coatings during the screen testing process to prevent coating failure, reduced production yields, and increased manufacturing costs. EP 0 785 173 discusses an apparatus comprising a capstan and a capstan belt having a Shore A hardness of 85.

US 2013/255397 A1 discusses a method and device for continuously testing the tensile strength of an optical fiber.

EP 0 514 858 A2 discusses a screening method for conducting tensile strength tests of an optical fiber, a wire or the like by applying a load thereto and an apparatus for carrying out the method.

US 4 997 276 A discusses an apparatus and method for determining the relative values of the properties of optical fibers, and, more particularly, to the continuous measurement of the variations in the elastic properties of the optical fiber buffer layer.

US 5 178 313 A discusses machines for handling filamentary or fiber optic cables and in particular to a pneumatic shoe and capstan design for paying out filament at high speed.

JP 2005 097051 A discusses a wire-drawing apparatus of optical fiber, where a capstan apparatus comprising a capstan belt which is made of foam material.

### SUMMARY

The present specification describes an integrated capstan for use in fiber processing and testing apparatus. The integrated capstan features a fiber-engaging material that provides a contact surface for fibers. The hardness of the contact surface is controlled to minimize transfer of stress to the fibers to prevent damage to fiber coatings or fiber splice junctions during conveyance or screen testing of fibers during production. The fiber-engaging material includes a resilient material and an optional capping layer. The resilient material may be a lightly crosslinked polyurethane gel. Capping layers include polymers formed by UV curing of aliphatic urethane diacrylate compounds or moisture curing of diisocyanate compounds.

The present specification extends to:
An integrated capstan comprising:
a capstan; and
a fiber-engaging material, said fiber-engaging material formed on the surface of said capstan, said fiber-engaging material having a hardness in the range from 40 Shore 00 to 70 Shore 00.

The present specification extends to:
An apparatus for processing an optical fiber comprising;
an integrated capstan according to claim 1; and a fiber conveyance pathway, said fiber conveyance pathway positioned adjacent to said integrated capstan such that when said optical fiber is directed along said fiber conveyance pathway, said optical fiber contacts said fiber-engaging material.

The present specification extends to:
A method for screen testing an optical fiber comprising:
drawing an optical fiber along a fiber conveyance pathway; and
directing said optical fiber around an integrated capstan, said integrated capstan comprising a capstan and a fiber-engaging material, said fiber-engaging material formed on the surface of said capstan, said fiber-engaging material having a hardness in the range from 40 Shore 00 to 70 Shore 00, said optical fiber contacting said fiber-engaging material.

Additional features and advantages will be set forth in the detailed description which follows, and in part will be readily apparent to those skilled in the art from the description or recognized by practicing the embodiments as described in the written description and claims hereof, as well as the appended drawings.

It is to be understood that both the foregoing general description and the following detailed description are merely exemplary, and are intended to provide an overview or framework to understand the nature and character of the claims.

The accompanying drawings are included to provide a further understanding, and are incorporated in and constitute a part of this specification. The drawings are illustrative of selected aspects of the present description, and together with the specification serve to explain principles and operation of methods, products, and compositions embraced by the present description. Features shown in the drawing are illustrative of selected embodiments of the present description and are not necessarily depicted in proper scale.

### BRIEF DESCRIPTION OF THE DRAWINGS

While the specification concludes with claims particularly pointing out and distinctly claiming the subject matter of the written description, it is believed that the specification will be better understood from the following written description when taken in conjunction with the accompanying drawings, wherein:
FIG. 1 schematically depicts a configuration of capstan assemblies used to convey or screen test optical fibers;
FIG. 2 schematically depicts a configuration of capstan assemblies with integrated capstans used to convey or screen test optical fibers;
FIG. 3 schematically depicts an integrated capstan and pinch belt according to one or more embodiments shown or described herein;
FIG. 4A schematically depicts a perspective cross-sectional view of an integrated capstan according to one or more embodiments shown or described herein;
FIG. 4B schematically depicts a front-view cross section of an integrated capstan according to one or more embodiments shown or described herein;
FIG. 4C schematically depicts an enlarged view of a portion of a cross-section of an integrated capstan according to one or more embodiments shown or described herein;
FIG. 5A schematically depicts a perspective cross-sectional view of an integrated capstan according to one or more embodiments shown or described herein;
FIG. 5B schematically depicts a front-view cross section of an integrated capstan according to one or more embodiments shown or described herein;
FIG. 5C schematically depicts an enlarged view of a portion of a cross-section of an integrated capstan according to one or more embodiments shown or described herein;
FIG. 6A shows a metal capstan having a channel.
FIG. 6B shows a metal capstan with a fiber-engaging material occupying a channel.
FIGS. 7A-7E depict steps in an illustrative process for forming a fiber-engaging material in the channel of a metal capstan.
FIG. 8 shows the time variation in shear storage modulus and shear loss modulus of a polyurethane resilient material in the channel of an integrated capstan at 90 °C.
FIG. 9 shows the time variation in shear storage modulus and shear loss modulus of a polyurethane resilient material in the channel of an integrated capstan at room temperature under conditions of tensile stress.
FIG. 10 shows the time dependence of the hardness of a polyurethane resilient material in the channel of an integrated capstan at room temperature.

The embodiments set forth in the drawings are illustrative in nature and not intended to be limiting of the scope of the detailed description or claims. Whenever possible, the same reference numeral will be used throughout the drawings to refer to the same or like feature.

### DETAILED DESCRIPTION

Reference will now be made in detail to embodiments of the apparatuses and methods for screen testing an optical fiber described herein, examples of which are illustrated in the accompanying drawings. Whenever possible, the same reference numerals will be used throughout the drawings to refer to the same or like parts.

As used herein, contact refers to direct contact or indirect contact. Direct contact refers to contact in the absence of an intervening material and indirect contact refers to contact through one or more intervening materials. Elements in direct contact touch each other. Elements in indirect contact do not touch each other, but do touch an intervening material. Elements in contact may be rigidly or non-rigidly joined. Contacting refers to placing two elements in direct or indirect contact.

As used herein, hardness of a material refers to hardness based on the Shore 00 scale as defined by the ASTM D2240-00 testing standard. Hardness may also be referred to herein as Shore hardness and may be reported using the notation XX Shore 00, where XX is the numerical hardness value based on the Shore 00 scale. Lower numerical values correspond to softer materials. A material with a hardness of 20 Shore 00, for example, is softer than a material with a hardness of 40 Shore 00. Hardness is measured with a durometer.

Optical fibers may be formed by drawing a thin glass fiber from a glass blank or preform. After the glass fiber is drawn from the preform, one or more coatings may be applied to the glass fiber to protect the glass and preserve the structural integrity of the optical fiber. To form optical fiber having an extended length, a plurality of optical fibers may be consecutively spliced together. To splice together a pair of optical fibers, the one or more coatings are stripped from the glass fibers near the ends that are to be joined together. The ends of the two glass fibers are joined together, and a recoat coating may be applied to the optical fiber to replace the stripped coatings.

The recoat coating applied to replace the stripped coatings may have a different compressive and shear modulus than the one or more coatings of the original optical fibers. In particular, the recoat coating may have a higher compressive modulus and a higher shear modulus than the one or more coatings on the original optical fibers. Because the recoat coating has a higher compressive modulus and shear modulus than the one or more coatings of the optical fibers, the recoat coating may exhibit less elastic deformation under compressive forces and shear forces imposed by the capstan and pinch belt. Because the original fiber coatings typically include a soft (low modulus) primary coating and because the recoat is a hard (high modulus) material, the recoat coating deforms less than the one or more coatings of the original optical fibers under the compressive and shear forces imposed by the capstan and pinch belt. As a result, stress may be placed on the optical fiber at the interface between the recoat coating and the one or more coatings of the original optical fibers. The stress placed on the optical fiber may lead to cohesive failure at the interface between the recoat coating and the one or more coatings of the optical fiber. The stress placed on the optical fiber may also lead to adhesive failure between the one or more coatings and the glass fiber. Additionally, the stress placed on the optical fiber may damage an outer surface of the one or more coatings. Accordingly, the stress placed on the optical fiber may damage the coatings of the optical fiber, leading to optical fiber being discarded, increasing manufacturing costs.

The capstans, apparatuses and methods described herein reduce the compressive and shear stress imposed on an optical fiber directed between at least one capstan and at least one pinch belt during a screen test of the optical fiber. Reducing the compressive and shear stress imposed on an optical fiber during a screen test of the optical fiber may reduce the likelihood of cohesive failure of the coatings of the optical fiber

The present specification provides an integrated capstan for optical fiber processing, systems for processing or testing optical fibers that include the integrated capstan, and methods for processing or testing optical fibers that include contacting an optical fiber to the integrated capstan. The integrated capstan includes a capstan and a fiber-engaging material integrated with the outer surface of the capstan. The fiber-engaging material receives an optical fiber during processing or testing of optical fibers and may also convey the optical fiber. The fiber contacts the fiber-engaging material. The fiber-engaging material includes a resilient material. The resilient material is a soft (low hardness or low modulus), flexible material that acts to reduce or relax stresses applied or transferred to the fiber during processing or testing. As a result, the failure rate of coatings on fibers and the occurrence of defects in fiber coatings are reduced.

The fiber-engaging material may be a single layer material or a multilayer material. In one embodiment, the fiber-engaging material includes a resilient material. The resilient material may be a polymer or gel and the optical fiber contacts the polymer or gel when conveyed during processing or testing. Contact of the optical fiber with the resilient material may be direct or indirect. Representative resilient materials include polyurethane materials or polyurethane gels.

In another embodiment, the fiber-engaging material includes a capping layer and a resilient material. The capping layer is contact with the resilient material and the optical fiber contacts the capping layer during processing or testing. Contact of the optical fiber with the capping layer may be direct or indirect. Contact of the capping layer with the resilient material may be direct or indirect. The surface of the capping layer may constitute a fiber-contact surface and can be designed to control or avoid adhesion or sticking of the fiber to the fiber-engaging material during positioning or conveyance of the fiber over the integrated capstan. The characteristics of the surface of the surface of the capping layer may differ from the characteristics of the surface of the resilient material. Representative capping materials include polyurethane prepolymer with moisture curable capability and urethane acrylate with UV curable capability.

In a further embodiment, the surface of the integrated capstan features a channel for receiving an optical fiber and the channel is filled with the fiber-engaging material. The channel may be partially filled, filled, or overfilled with the fiber-engaging material.

In other embodiments, the integrated capstan may include an elastic material in contact with the fiber-engaging material. The elastic material may be a removable material and may function to protect the fiber-engaging material. The elastic material may be in direct contact with the fiber-engaging material. In one embodiment, the fiber-engaging material includes a capping layer in contact with a resilient material and the elastic material directly contacts the capping layer.

The present specification further provides an apparatus for processing or screen testing an optical fiber. The apparatus includes the integrated capstan with capstan and fiber-engaging material as described herein. During operation, the apparatus directs an optical fiber to the integrated capstan for positioning on or conveyance by the fiber-engaging material. The apparatus may include one or more pinch belts for pressing the optical fiber on to one or more integrated capstans during conveyance or screen testing. The optical fiber may be in contact with one or more pinch belts and/or one or more integrated capstans during processing or screen testing. The optical fiber may be stationary or in motion during processing or screen testing while in contact with one or more integrated capstans. The apparatus may further include additional processing units such as a furnace for heating an optical fiber preform, a draw tower, heating and cooling stages for processing the glass portion of the optical fiber, and coating stations for applying one or more coatings to the glass portion of the optical fiber. The one or more coatings may include a primary coating and/or secondary coating.

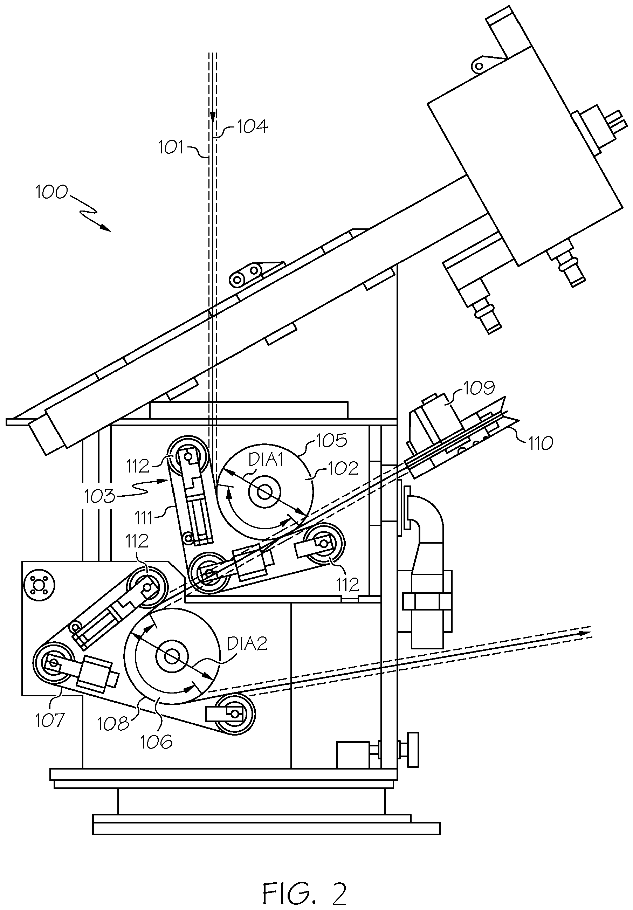

Referring to FIG. 2, one embodiment of a screen testing apparatus 100 for screen testing an optical fiber is schematically depicted. The screen testing apparatus 100 generally includes a fiber conveyance pathway 101 that extends through the screen testing apparatus 100. The fiber conveyance pathway 101 of the screen testing apparatus 100 defines a pathway over which an optical fiber is directed during the screen test. The screen testing apparatus 100 generally includes at least a first integrated capstan 102 positioned adjacent to a fiber conveyance pathway 101 and at least a first pinch belt 103 positioned adjacent to the fiber conveyance pathway 101 opposite the first integrated capstan 102. The first integrated capstan 102 and the first pinch belt 103 are positioned so that the fiber conveyance pathway 101 is positioned between the first integrated capstan 102 and the first pinch belt 103. As an optical fiber 104 is directed over the fiber conveyance pathway 101, the first pinch belt 103 impinges the optical fiber 104 between the first pinch belt 103 and the first integrated capstan 102.

The first integrated capstan 102 has a first diameter DIA1, and an outer circumference 105. The outer circumference 105 of the first integrated capstan 102 is positioned adjacent to the fiber conveyance pathway 101 so that the optical fiber 104 directed over the fiber conveyance pathway 101 engages the outer circumference 105 of the first integrated capstan 102.

The screen testing apparatus 100 may optionally include a second integrated capstan 106 positioned adjacent to the fiber conveyance pathway 101 and a second pinch belt 107 positioned adjacent to the fiber conveyance pathway 101. Similar to the first integrated capstan 102 and the first pinch belt 103, the second integrated capstan 106 and the second pinch belt 107 may be positioned so that the fiber conveyance pathway 101 is positioned between the second integrated capstan 106 and the second pinch belt 107. As the optical fiber 104 is directed over the fiber conveyance pathway 101, the second pinch belt 107 impinges the optical fiber 104 between the second pinch belt 107 and the second integrated capstan 106.

The second integrated capstan 106 may have a second diameter DIA2, and an outer circumference 108. The outer circumference 108 of the second integrated capstan 106 is positioned adjacent to the fiber conveyance pathway 101 so that an optical fiber 104 directed over the fiber conveyance pathway 101 engages the outer circumference 108 of the second integrated capstan 106.

The screen testing apparatus 100 may optionally include a load cell 109 and a pulley 110 positioned adjacent to the fiber conveyance pathway 101. The load cell 109 and the pulley 110 are positioned adjacent to the fiber conveyance pathway 101 between the first integrated capstan 102 and the second integrated capstan 106. The pulley 110 may be positioned adjacent to the fiber conveyance pathway 101 so that the pulley contacts the optical fiber 104 directed over the fiber conveyance pathway 101. The load cell 109 may be coupled to the pulley 110 so that the load cell detects a tension in the optical fiber 104 through the contact between the optical fiber 104 and the pulley 110.

To rotate the first integrated capstan 102 and the second integrated capstan 106, the first integrated capstan 102 may be connected to a first driveshaft (not depicted) and the second integrated capstan 106 may be connected to a second driveshaft (not depicted) that is driven independent of the first driveshaft. The first driveshaft and the second driveshaft may be driven by power sources which may include without limitation, electric motors, pneumatically driven spindles, and the like.

The first integrated capstan 102 and the second integrated capstan 106 apply a tensile stress on the optical fiber 104 directed over the fiber conveyance pathway 101. To apply a tensile stress on the optical fiber 104, the first integrated capstan 102 and the second integrated capstan 106 may be rotated at different rotational speeds. More specifically, the second integrated capstan 106 may be rotated at a higher rotational speed than the first integrated capstan 102. As a result of the higher rotational speed of the second integrated capstan 106, a portion of the optical fiber 104 between the first integrated capstan 102 and the second integrated capstan 106 on the fiber conveyance pathway 101 will be under tension.

Alternatively, to apply a tensile stress to the optical fiber, the diameter DIA2 of the second integrated capstan 106 may be selected to be larger than the diameter DIA1 of the first integrated capstan 102. When the rotational speed of the second integrated capstan 106 is the same or higher than the rotational speed of the first integrated capstan 102, and the diameter DIA2 of the second integrated capstan 106 is greater than the diameter DIA1 of the first integrated capstan 102, a linear speed of the outer circumference 108 of the second integrated capstan 106 will be higher than a linear speed of the outer circumference 105 of the first integrated capstan 102. As a result of the higher linear speed of the outer circumference 108 of the second integrated capstan 106, the portion of the optical fiber 104 between the first integrated capstan 102 and the second integrated capstan 106 on the fiber conveyance pathway 101 will be under tension.

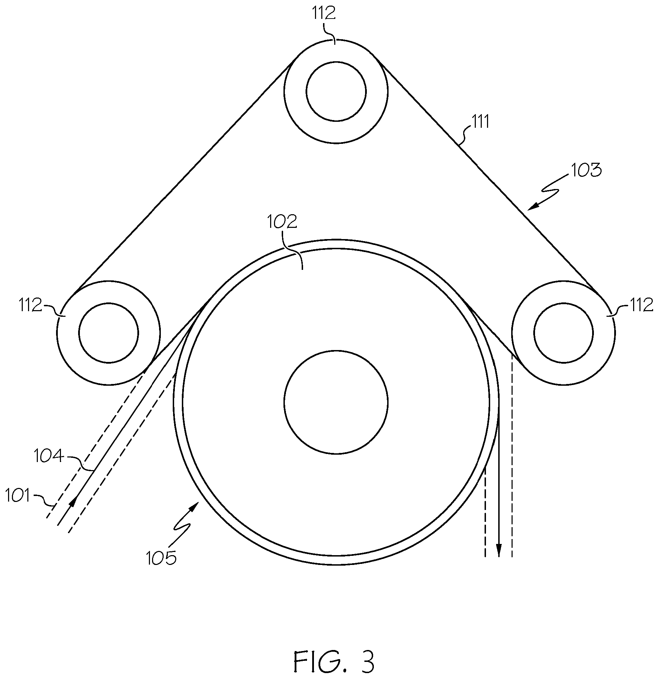

Referring now to FIGS. 2 and 3, to isolate the tension applied to the optical fiber 104 by the first integrated capstan 102 and the second integrated capstan 106, the first pinch belt 103 impinges the optical fiber 104 between the first pinch belt 103 and the first integrated capstan 102. In embodiments, the first pinch belt 103 may include a first belt 111 and a plurality of idler pulleys 112 that are positioned adjacent to the fiber conveyance pathway 101. The first belt 111 is positioned around the plurality of idler pulleys 112 so that the first belt 111 impinges the optical fiber 104 directed over the fiber conveyance pathway 101 between the first belt 111 and the first integrated capstan 102. Accordingly, the first pinch belt 103 applies a compressive force to the optical fiber 104 between the first pinch belt 103 and the first integrated capstan 102 to isolate the tension applied to the optical fiber 104 between the first integrated capstan 102 and the second integrated capstan 106. In embodiments, the position of the plurality of idler pulleys 112 may be adjustable, such that a tension in the first belt 111 and, accordingly, the compressive force applied to the optical fiber 104, may be adjusted. To adjust the position of the idler pulleys 112, the idler pulleys may be coupled to the screen testing apparatus by actuators, such as pneumatic devices, electric motors, and the like. While reference has been made hereinabove to the configuration of the first pinch belt 103 and the first integrated capstan 102, it should be understood that the second pinch belt 107 and the second integrated capstan 106 may likewise include a plurality of adjustable idler pulleys 112 to isolate the tension in the optical fiber 104 between the first integrated capstan 102 and the second integrated capstan 106.

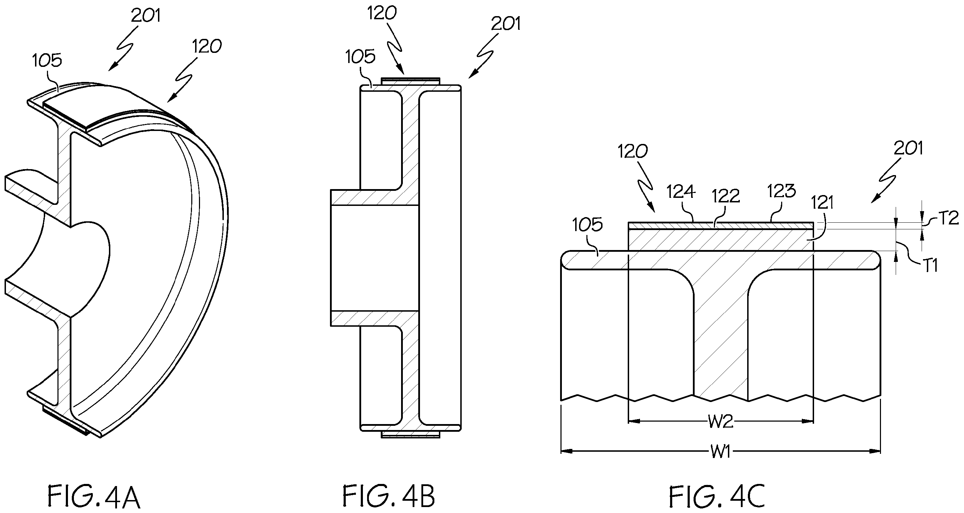

To reduce the compressive and shear stress placed on the optical fiber 104 during the screen testing process, various embodiments of capstans which may be used as the first integrated capstan 102 and/or the second integrated capstan 106 are described herein. Referring to FIGS. 4A, 4B, and 4C, one embodiment of an integrated capstan 201 is depicted. The integrated capstan 201 is generally cylindrical, and has a diameter DIA1, a width W1, and an outer circumference 105. The outer circumference 105 of the first integrated capstan 102 includes a fiber-engaging material 120 extending around the outer circumference 105 of the integrated capstan 201. The fiber-engaging material 120 of the integrated capstan 201 may be the portion of the outer circumference 105 of the integrated capstan 201 that engages the optical fiber 104 directed over the fiber conveyance pathway 101. In embodiments, the fiber-engaging material 120 may have a width W2 that is greater than or equal to about ten times a diameter of an optical fiber 104 directed over the fiber conveyance pathway 101.

In this embodiment, the fiber-engaging material 120 includes resilient material 121 and capping material 123 with surface 124. Resilient material 121 and capping material 123 may be configured as layers. Resilient material 121 may be referred to herein as a layer of resilient material or resilient layer. Capping material 123 may be referred to herein as a layer of capping material or capping layer. The resilient material 121 may be positioned over the outer circumference 105 of the integrated capstan 201, extending around the outer circumference 105 of the integrated capstan 201. The resilient material 121 of the fiber-engaging material 120 has a thickness T1. In embodiments, the thickness T1 may be greater than or equal to about 1 mm and less than or equal to about 25 mm, or greater than or equal to about 3 mm and less than or equal to about 20 mm, or greater than or equal to about 5 mm and less than or equal to about 17 mm, or greater than or equal to about 7 mm and less than or equal to about 13 mm, or about 10 mm.

The resilient material 121 of the fiber-engaging material 120 may be selected to have a desired hardness and a desired compressive modulus and shear modulus to reduce the compressive and shear stresses on the optical fiber 104 contacting the fiber-engaging material 120. In embodiments, the resilient material 121 is selected to have a durometer hardness of less than 90 Shore 00, or less than 80 Shore 00, or less than 70 Shore 00, or less than 60 Shore 00, or less than 50 Shore 00, or less than 40 Shore 00, or in the range from 30 Shore 00 - 80 Shore 00, or in the range from 35 Shore 00 - 75 Shore 00, or in the range from 40 Shore 00 - 70 Shore 00, or in the range from 40 Shore 00 - 65 Shore 00, or in the range from 45 Shore 00 - 60 Shore 00, or in the range from 45 Shore 00 - 55 Shore 00. The hardness values listed herein for the resilient material apply to fiber-engaging materials that include a resilient material and a capping material as well as to fiber-engaging materials that include a resilient material without a capping material.

In embodiments, the resilient material 121 may be an isotropic material, where the hardness of the material correlates to a compressive modulus and a shear modulus of the material. More specifically, a higher hardness value for resilient material 121 may correlate with a higher compressive modulus and a higher shear modulus of resilient material 121. Conversely, a lower hardness value for the resilient material 121 may correlate to a lower compressive modulus and a lower shear modulus of resilient material 121. A low hardness value, and consequently a low compressive modulus and shear modulus for resilient material 121 may reduce the compressive and shear stress in the optical fiber 104 in contact with the fiber-engaging material 120.

The fiber-engaging material 120 of the integrated capstan 201 may further include capping layer 123 positioned over resilient material 121. In embodiments, the capping layer 123 may extend laterally in a width direction to cover resilient material 121 of the fiber-engaging material 120. The capping layer 123 may have a thickness T2. The thickness T2 of capping layer 123 may be in the range from 0.1 µm to 10 µm, or in the range from 0.25 µm to 5.0 µm, or in the range from 0.5 µm to 4.0 µm, or in the range from 0.75 µm to 3.0 µm, or in the range from 1.0 µm to 2.0 µm.

Capping layer 123 may be selected to have a desired hardness. Capping layer 123 may be selected to have a hardness within ±10 Shore 00 above or below the hardness of resilient material 121, or within ±5 Shore 00 above or below the hardness of resilient material 121, or within the range from 40 Shore 00 - 100 Shore 00, or within the range from 50 Shore 00 - 90 Shore 00, or within the range from 55 Shore 00 - 85 Shore 00, or within the range from 60 Shore 00 - 80 Shore 00.

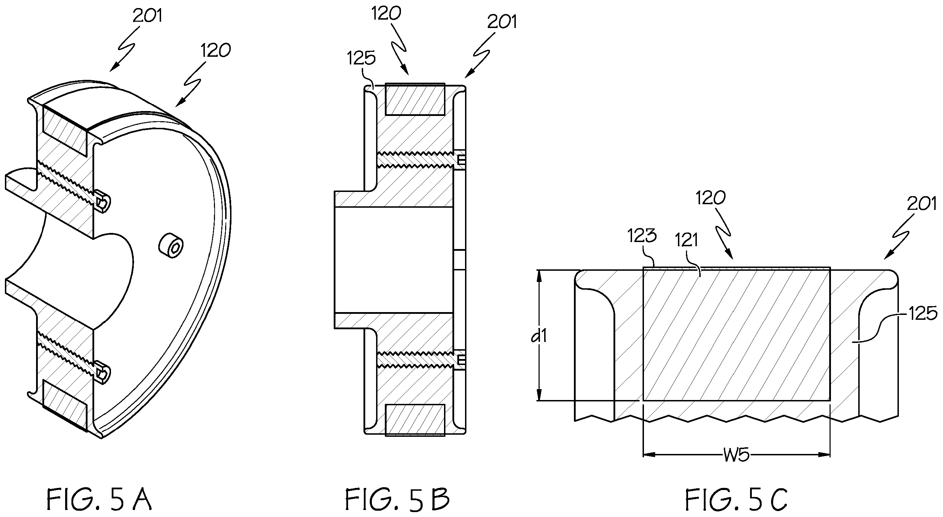

Referring now to FIGS. 5A, 5B, and 5C, another embodiment of the integrated capstan 201 is schematically depicted. In this embodiment, the integrated capstan 201 includes a channel 125 extending radially inward from the outer circumference 105 of the integrated capstan 201. The channel 125 may have a depth d1 extending radially inward from the outer circumference 105 of the integrated capstan 201, and a width W3 extending across the outer circumference 105 of the integrated capstan 201. The depth d1 may be selected to be greater than or equal to 1 mm and less than or equal to 12 mm. In an alternative embodiment, the depth d1 may be selected to be greater than or equal to about 1 mm and less than or equal to about 5 mm. The resilient material 121 of the fiber-engaging material 120 may be positioned partially or completely within the channel 125 of the integrated capstan 201. Optional capping layer 123 may be positioned over resilient material 121 and may extend over the full width of channel 125 to enclose channel 125. The materials and hardness of resilient material 121 and capping layer 123 are as described hereinabove and in connection with FIGS. 4A-4C.

In one embodiment, a vacuum suction may be applied to the channel 125 to retain the fiber-engaging material 120 within the channel 125. The vacuum suction may be applied to the channel 125 by mechanisms including, without limitation, a one-way valve positioned on the integrated capstan 201, the one-way valve in fluid communication with the channel 125. As the integrated capstan 201 rotates, the vacuum suction may counteract a centrifugal force to retain the inner layer 121 within the channel 125. By positioning the fiber-engaging material 120 within a channel 125 and utilizing a force, such as a vacuum suction, to retain the fiber-engaging material 120 within the channel 125, the fiber-engaging material 120 may be prevented from coming free from the surface of integrated capstan 201 by the centrifugal force caused by rotation.

Integration of a fiber-engaging material with the capstan reduces stresses on the fiber and fiber coatings as the fiber is conveyed over the capstan during processing or screen testing. High levels of stress, sufficient to damage coatings and cause failures at splice junctions, arose in prior art systems because fibers traversed capstans with hard surfaces. Conventional capstans are metals (e.g. aluminum) with uncoated surfaces. The integrated capstans described herein reduce stresses by providing fiber-engaging materials with fiber-contact surfaces having low hardness. The fiber-engaging materials are integrated on the surface of capstans and are especially beneficial when used with capstans, such as metal capstans, that have high hardness surfaces. The fiber-engaging material provides a cushion capable of absorbing or dissipating stresses that might otherwise develop in the fiber during processing or screen testing.

It has been determined herein that careful selection of the fiber-engaging material is necessary to achieve the objective of low stress generation at fiber coatings or splice junctions during processing or screen testing. Although it is desirable to lower the hardness of the fiber-contact surface to below the hardness of metal, the fiber-engaging material must be rigid enough to provide sufficient resistance, when contacted by the fiber, to prevent the fiber from displacing the fiber-engaging material to directly contact the underlying metal surface of the capstan. Conveyance of the fiber through a fiber processing or screen testing apparatus is driven by tension. The drive tension provides a force that induces motion of the fiber toward contact surfaces of capstans and other guiding or directional units in the apparatus. High drive tensions are generally preferred because the permit faster draw speeds and more cost-efficient manufacturing. In order to resist the tendency of the drive tension to force the fiber onto the underlying metal surface of the present integrated capstans, the fiber-engaging material must have hardness sufficient to counteract the drive tension so that the fiber remains in contact with the fiber-engaging material. At the same time, the fiber-engaging material must manage the level of stress at the fiber coating and splice junctions to prevent coating defects and failures.

Tests with commercial polymers with the lowest available hardness (~96 Shore 00) revealed that the polymers were adequate to prevent direct contact of the fiber with the underlying metal surface of the capstan, but were inadequate to prevent coating defects and failures. New formulations were thus developed to provide fiber-engaging materials having performance superior to the performance of existing commercial materials. The new formulations are now described.

In one embodiment, the resilient material of the present fiber-engaging materials is a polyurethane gel. The polyurethane gel is formed from a reaction of an isocyanate compound and a hydroxy compound or from a reaction of compounds that include an isocyanate group and a hydroxy group. The hydroxy compound may be a monofunctional or multifunctional hydroxy compound. A monofunctional hydroxy compound is a compound that contains a single reactive hydroxy group. A multifunctional hydroxy compound is a compound that includes two or more reactive hydroxy groups (e.g. dihydroxy compound, trihydroxy compound etc.). In one embodiment, the reactive hydroxy group(s) is (are) terminal groups. Multifunctional compounds may also have mixed functionality (e.g. include two or more different types of reactive functional groups as terminal or pendent groups within the same molecule). Reaction of an isocyanate group with a hydroxyl group forms a urethane linkage.

Representative isocyanate compounds include polymers with one or more isocyanate groups. The isocyanate groups are terminal groups. Terminal groups are bonded to the end(s) of the polymer. The isocyanate groups may be directly bonded to the polymer or bonded to the polymer via a linkage group. The linkage group has a site of bonding with the isocyanate group and a site of bonding to the polymer. The linkage group may also be referred to herein as a linking group. Isocyanate groups bonded to the polymer through a linkage group may be referred to herein as linked isocyanate groups.

Exemplary polymers include polyether compounds with two terminal isocyanate groups. The isocyanate groups may be directly bonded to the polymer or bonded through a linkage group. In one embodiment, the linkage group includes an aromatic group (e.g. phenyl or phenyl-containing group). Aromatic linkages may include a 4,4'-methylenebisphenyl fragment. In another embodiment, the isocyanate group is bonded to the polymer through a urethane linkage.

Representative hydroxy compounds include polymers with one or more hydroxy groups. The hydroxy groups may be terminal or pendent and may be directly bonded to the polymer or bonded to the polymer through a linkage group. Example hydroxy compounds include polyalkyene or polyalkyl-di-ene polymers with two or more terminal or pendent hydroxy groups, such as hydroxy-terminated polybutadiene.

The reaction to form the resilient material may include reactions between one or more isocyanate compounds and one or more hydroxy compounds. The reaction may occur in solution (or suspension) and may be thermally driven. The reaction may include a curing step and be performed in the presence of a catalyst. Representative catalysts include dimethylbis [(1-oxoneodecyl)oxy] stannate.

The hardness of the resilient material can be controlled through selection of the isocyanate and hydroxyl reactant compounds. The resilient material may include hard block segments and soft block segments. Hard block segments impart rigidity to the resilient material and act to increase hardness. Soft block segments are more flexible than hard block segments and act to decrease hardness. Control of the hardness of the resilient material can be achieved by balancing the relative proportion of hard block and soft block segments in the resilient material. The relative proportions of hard and soft block segments can be controlled by the concentration of reactant molecules that contribute hard and soft block segments and/or through the number of hard and soft block segments per reactant molecule (e.g. molecular weight or number of repeat units of hard or soft block segments in the reactant molecules).

Compounds with aliphatic groups (along the main chain or as linking groups) (e.g. alkylene or alkyl-di-ene) or oxygenated groups (e.g. ether groups, oxyalkylene groups along the main chain or as linking groups) provide soft block segments in the molecules of the resilient material and tend to decrease the hardness of the resilient material provide. Compounds with aromatic groups (along the main chain or as linking groups) (e.g. phenyl groups, phenyl-containing groups) provide hard block segments in the molecules of the resilient material and tend to increase the hardness of the resilient material.

In order to adequately protect fibers from coating damage or failure, the hardness of the resilient material is controlled according to the following embodiments: less than 90 Shore 00, or less than 80 Shore 00, or less than 70 Shore 00, or less than 60 Shore 00, or less than 50 Shore 00, or less than 40 Shore 00, or in the range from 30 Shore 00 - 80 Shore 00, or in the range from 35 Shore 00 - 75 Shore 00, or in the range from 40 Shore 00 - 70 Shore 00, or in the range from 40 Shore 00 - 65 Shore 00, or in the range from 45 Shore 00 - 60 Shore 00, or in the range from 45 Shore 00 - 55 Shore 00.

The degree of crosslinking in the resilient material is another factor that influences hardness. Hardness generally increases as the resilient material becomes more crosslinked. Crosslinking is controlled by the number of branching points in the polymer molecule, which in turn is controlled by the number of reactive functional groups along the polymer chain or in the compounds used as starting materials for the polymer. As noted hereinabove, for example, polyurethane gels can be formed by reacting isocyanate compounds with hydroxy compounds. Crosslinking occurs when the hydroxy compound includes three or more hydroxy groups, where the degree (extent) of crosslinking is controlled by the average number of hydroxy groups in the molecules of the hydroxy compound. As the number of hydroxy groups per molecule in the hydroxy compound increases, the crosslinking becomes more extensive and the hardness of the polyurethane gel increases.

In one embodiment, the resilient material is a polyurethane material formed by a reaction of a polyether compound with diisocyanate groups. The isocyanate content of the polyether isocyanate compound may be expressed in terms of the weight percent (wt%) of isocyanate groups in the compound. The wt% of isocyanate groups is computed as the ratio of the molecular weight of all isocyanate groups in the compound to the total molecular weight of the compound. For purposes of calculating wt%, the contribution of isocyanate groups to the molecular weight is based on the isocyanate group (-N=C=O) independent of any linking groups and the ratio is taken as an average over all molecules of the polyether isocyanate compound(s). An analogous definition can be used to express the hydroxy content of multifunctional hydroxy compound(s).

To maintain a hardness for the resilient material in a range that minimizes damage to fiber coatings, it has been determined that the degree of crosslinking of the resilient material should be kept low. Commercially available polyurethane materials have a high degree of crosslinking and are accordingly rigid with hardness values above the ones stated herein that are conducive to processing fibers without damaging fiber coatings. Such materials are made from isocyanate compounds with an isocyanate content of 20 wt% or more. In embodiments herein, the reinforcing material is a polyurethane material made from a polyether isocyanate compound having an isocyanate content of less than 15 wt%, or less than 12 wt%, or less than 10 wt%, or in the range from 2 wt% - 15 wt%, or in the range from 4 wt% - 12 wt%, or in the range from 6 wt% - 10 wt%. In one embodiment, polyether isocyanate compounds having the stated isocyanate content with linkages that include aromatic groups. The polyurethane reinforcing material may be made from reactions of the polyether isocyanate compounds having the above stated isocyanate content with a bifunctional or multifunctional hydroxy compound.

The degree of crosslinking of the resilient material can also be influenced by the relative amounts of isocyanate compounds and hydroxy compounds. The molar proportion of isocyanate compounds to hydroxy compounds may be in the range from 0.50 to 1.50, or in the range from 0.70 to 1.20, or in the range from 0.80 to 1.05, or in the range from 0.85 to 0.95.

The capping material is a polymer that may be formed by UV curing or moisture curing. In one embodiment, the capping material is obtained through UV curing of a formulation that includes an acrylate compound. The acrylate compound may be monofunctional of multifunctional. The acrylate compound may include urethane linkages. In one embodiment, the acrylate compound is a bifunctional acrylate compound with terminal acrylate groups. Exemplary acrylate compounds include diacrylates such as aliphatic diacrylates or urethane diacrylates. Urethane diacrylates are diacrylate compounds that include urethane linkages. The urethane linkages may be separated by aliphatic groups. The acrylate compound may be an aliphatic acrylate compound. The aliphatic acrylate compound may include urethane linkages. The aliphatic acrylate compound may lack aromatic groups.

To improve the cohesiveness of the fiber-engaging material, it is desirable to select a capping material that is chemically compatible with the resilient material. Chemical compatibility can be promoted by including linkages or segments in the molecules of the capping material that have high affinity with the molecules of the resilient material. High affinity can result, for example, from strong intermolecular association or hydrogen bonding. In one embodiment, the resilient material is a polyurethane material and the capping material is a polymer that includes urethane linkages. The UV-curable formulation may include a combination of diacrylate (or multifunctional acrylate) compounds and a photoinitiator. Other additives (such as solvents, chain transfer agents or chain termination agents) may also be present.

Capping materials include materials formed from moisture-curable multifunctional isocyanate compounds. The moisture-curable multifunctional isocyanate compounds may be terminal diisocyanate compounds. One example of a capping material is the moisture-cured product of 4,4'-diphenylmethane diisocyanate. 4,4'-diphenylmethane diisocyanate includes two terminal isocyanate groups, each of which can react with water to convert the terminal isocyanate functionality to hydroxy or acid functionality. The converted terminal groups can react with each other in a condensation reaction to release water and link molecules to form extended chains (oligomers and ultimately polymers).

In the course of testing resilient materials within the stated hardness ranges, a further complication was discovered. Specifically, many of the resilient materials with suitable hardness values were tacky. That is, when cured to form a solid coating on the capstan, the surface of the resilient material was sticky to the touch. A fiber-engaging material having a tacky surface is undesirable because it may adhere the fiber as the fiber passes over and contacts the fiber-engaging material during processing. Adhesion of the fiber may alter the fiber conveyance pathway and render the process unstable. The force associated with the motion of conveyance may also cause the fiber to tear or strip the fiber-engaging material.

To avoid complications arising from tacky fiber-contact surfaces, the capping layer may have a non-tacky surface. In this embodiment, the capping layer adheres to the tacky surface of a resilient material and presents a non-tacky surface to the fiber. The fiber engages the non-tacky fiber-contact surface during processing or testing without adhering to or damaging the fiber-contact surface.

As noted hereinabove, a protective material may be placed over the fiber-engaging material. The protective material may be an elastic material and the fiber-engaging material may include a capping layer with a non-tacky surface in contact with a resilient material having a tacky surface. It is desirable to have the ability to remove, replace, or exchange the protective material. Direct contact of the protective material with a tacky surface of a resilient material may cause the protective material to adhere to the tacky surface and lead to damage of the resilient material when removing the protective material. Damage to the protective material can be avoided by including a capping layer with a non-tacky surface between the protective material and the tacky surface of the resilient material. Direct contact of the protective material with the non-tacky surface permits removal or replacement of the protective material without damage to either the protective material or fiber-engaging material.

The present specification further extends to apparatuses for processing or testing an optical fiber. The apparatus includes the integrated capstan disclosed herein. In one embodiment, the apparatus includesan integrated capstan, where the integrated capstan includes a capstan and a fiber-engaging material formed on the surface of the capstan and the fiber-engaging material has a hardness as disclosed herein, such as a hardness in the range from 40 Shore 00 to 70 Shore 00.

The apparatus may also include a fiber conveyance pathway positioned adjacent to the integrated capstan such that when the optical fiber is directed along the fiber conveyance pathway, the optical fiber contacts the fiber-engaging material. Contact of the optical fiber with the fiber-engaging material may be direct or indirect. The optical fiber may contact an elastic material and the elastic material may contact the fiber-engaging material.

The apparatus may also include a pinch belt positioned adjacent to the fiber conveyance pathway with the fiber conveyance pathway extending between at least a portion of the pinch belt and the fiber-engaging material, where the pinch belt is engagable with the fiber-engaging material such that when the optical fiber is directed over the fiber conveyance pathway, the optical fiber is impinged between the pinch belt and the fiber-engaging material.

The present specification further extends to method for processing or testing an optical fiber. The testing may include screen testing. In one embodiment, the method includes drawing an optical fiber along a fiber conveyance pathway and directing the optical fiber around an integrated capstan, where the integrated capstan includes a capstan and a fiber-engaging material formed on the surface of the capstan, where the fiber-engaging material has a hardness in the range from 40 Shore 00 to 70 Shore 00 and the optical fiber contacts the fiber-engaging material. The method may also include directing the optical fiber between a pinch belt positioned adjacent to the fiber conveyance pathway and the fiber-engaging material, and contacting the pinch belt with the optical fiber.

### Examples

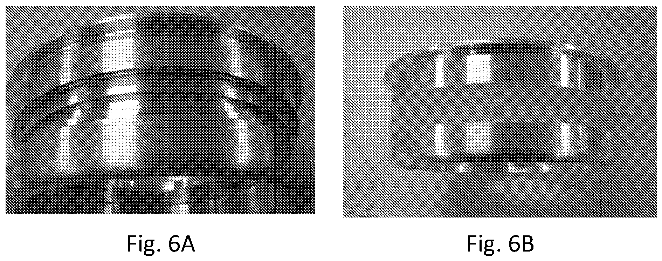

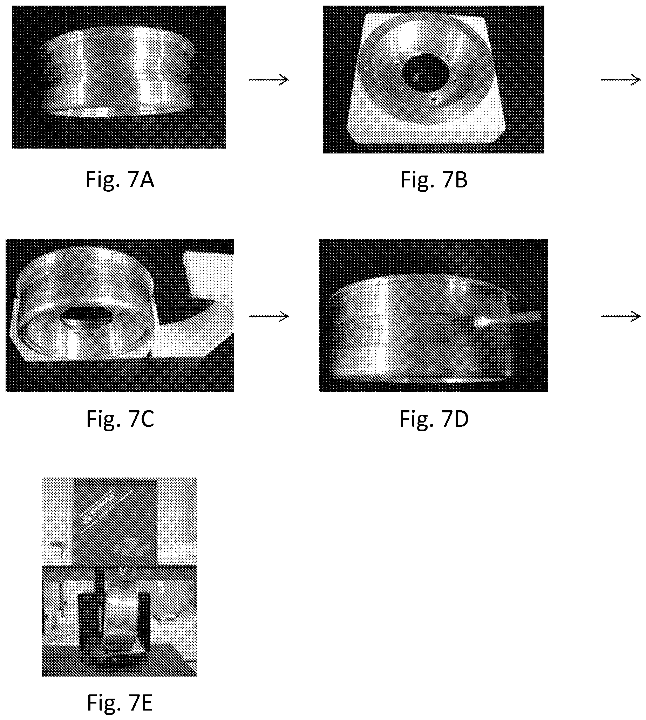

FIG. 6A shows an aluminum capstan having a central channel. FIG. 6B shows the same capstan when the channel is filled with a polyurethane gel. FIGS. 7A-7E show one process for filling the channel with a polyurethane gel. FIG. 7A shows an aluminum capstan with an unfilled channel. The capstan is inserted into a plastic mold in FIG. 7B and an uncured polyurethane gel formulation is injected into the groove through an injection hole. The injection hold can be located on the mold or machined into the capstan. While configured in the mold, the polyurethane gel formulation is cured. In the example shown, curing is accomplished by heating the capstan in the mold for 2 hours at 90 °C. After curing, the mold is removed (FIG. 7C). A capping layer is applied in FIG. 7D. In the example shown, the capping layer is applied to the surface of the cured polyurethane material in liquid form by brushing or spraying. Curing of the liquid capping layer formulation to form a solid capping layer is shown in FIG. 7E.

A representative formulation from which a polyurethane-type resilient material was made is now described. The formulation provided the resilient material shown in FIGS. 6B and 7C. The formulation included an isocyanate component and a hydroxy component. The isocyanate component was an MDI-terminated polyether prepolymer based on polypropylene ether glycol (PPG) having the general formula 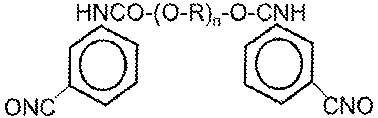 where R is a propylene group. The prepolymer included terminal groups derived from MDI (4,4'-methylenebis(phenyl isocyanate)) and had an isocyanate content of ~8 wt%. The MDI-terminated polyether prepolymer was obtained from Bayer Co. (Baytec MP-080) and was in the form of a liquid having a viscosity of ~2500 cP at 25 °C. The isocyanate component of the resilient material formulation consisted exclusively of the MDI-terminated polyether prepolymer (100 parts by weight).

The hydroxy component included the following compounds in the amounts indicated Table 1:

**Table 1**

| **Hydroxy Component of Polyurethane Resilient Material Formulation** | |
|---|---|
| Compound | Parts by Weight |
| hydroxy-terminated polybutadiene resin | 90 |
| diisodecyl phthalate | 90 |
| 1,4-butanediol | 1.65 |
| dimethylbis[(1-oxoneodecyl)oxy]stannate | 0.05 |

The hydroxy-terminated polybutadiene resin had the formula: 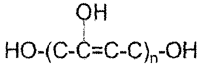 with a number average molecular weight of ~2800 g/mol, hydroxy functionality of ~2.5, and an OH content of ~0.84 meq/g. The hydroxy-terminated polybutadiene resin was obtained from Sartomer (Poly bd R45HTLO). Diisodecyl phthalate is a plasticizer and was obtained from Ashland Co. 1,4-butandiol was added for finer control of the hardness of the polyurethane-type resilient material and was obtained from Alfa Aesar Co. Dimethylbix[(1-oxoneodecyl)oxy]stannate is a catalyst and was obtained from Momentive Co.

The isocyanate component and hydroxy component were combined in a 1.5:5 ratio by weight to provide a formulation for the resilient material. The relative amounts of each compound in the formulation are summarized in Table 2:

**Table 2**

| **Polyurethane Resilient Material Formulation** | |
|---|---|
| Compound | Parts by Weight |
| MDI-terminated polyether prepolymer | 150 |
| hydroxy-terminated polybutadiene resin | 450 |
| diisodecyl phthalate | 450 |
| 1,4-butanediol | 8.25 |
| dimethylbis[(1-oxoneodecyl)oxy]stannate | 0.25 |

Curing of ~400 g of the formulation at 90 °C in a cup having a thickness of ~10 cm was complete in ~4 hours and provided a polyurethane resilient material having a hardness of 50 Shore 00 (as measured by a Shore 00 durometer) and a tacky surface.

A representative UV-curable formulation for forming a capping material is shown in Table 3:

**Table 3**

| **UV-Curable Capping Material Formulation** | |
|---|---|
| Compound | Parts by Weight |
| aliphatic urethane diacrylate | 100 |
| 1,6-hexanediol diacrylate | 40 |
| 1-hydroxy-cyclohexylphenylketone | 3 |

The aliphatic urethane diacrylate is available under the trade name Ebecryl 230 and was obtained from CYTEC Surface Specialties. The aliphatic urethane diacrylate had a viscosity of ~44014 cP at 25 °C. 6-hexandiol diacrylate was also obtained from CYTEC Surface Specialies (HDODA). 1-hydroxy-cyclohexylphenylketone is a UV-activated photoinitiator that was obtained from CYTEC (Additol cpk). The capping material can be formed by curing the formulation for 30 seconds with a 400 W mercury lamp (Dymax) to obtain a film with thickness in the range from 1-3 mm. The capping layer formed from the formulation listed in Table 3 is the capping layer depicted in FIG. 7E.

A representative moisture-curable formulation for forming a capping material is shown in Table 4:

**Table 4**

| **Moisture-Curable Capping Material Formulation** | |
|---|---|
| Compound | Parts by Weight |
| 4,4'-diphenylmethane diisocyanate | 100 |
| cyclohexane | 30-50 |

The formulation fully cures overnight in air at room temperature.

Fiber-engaging materials using the resilient material and capping materials prepared from the formulations listed in Tables 2-4 were formed in the channel of several capstans. Integrated capstans using the polyurethane resilient material formed from the formulation of Table 2 and capping materials formed from each of the formulations listed in Tables 3 and 4 were prepared and tested for compatibility with a continuous fiber manufacturing process. The capstans were aluminum and had channels with a width of 25 mm and depth of 12.5 mm. The resilient material was applied by placing the capstan in a mold, injecting the formulation of Table 2 into the channel, and thermally curing the formulation for 4 hours at 90 °C. The cured polyurethane resilient material filled the channel. After curing, the capping material formulation was applied as a liquid solution to the surface of the cured polyurethane resilient material. Separate capstans were prepared using the UV-curable capping material formulation of Table 3 and the moisture-curable capping material formulation of Table 4. The UV-curable capping material formulation was cured with UV light having a wavelength of 260-600 nm, intensity of 225 W/cm², for 30 sec to provide a capping material with a thickness of 1-3 µm. The moisture-curable capping material formulation was cured in air for at least 12 hours at room temperature.

The integrated capstans were tested in a fiber processing system under typical draw conditions. Fibers with splice junctions were passed over the integrated capstans at draw speeds of over 30 m/s and were subsequently inspected for damage. No damage to the fiber coating at the splice junction was detected.

Further tests were completed on the polyurethane resilient material prepared from the formulation listed in Table 2. The polyurethane resilient material was formed in the channel of a capstan as described above (injection of formulation into the channel of a capstan situated in a mold followed by thermal curing). The polyurethane resilient material filled the channel (depth ~12 mm and width ~25 mm). No capping material was applied in these tests. The polyurethane resilient material in the absence of a capping material was tested for stability over time. In comparative tests with commercial polyurethane materials, it was noticed that in addition to being too high to prevent damage to fiber coatings, the hardness of the commercial polyurethane materials was not stable over time or at elevated temperatures. The variability in hardness was attributed to instability in the commercial polyurethane materials due to incomplete reaction or crosslinking of the formulation.

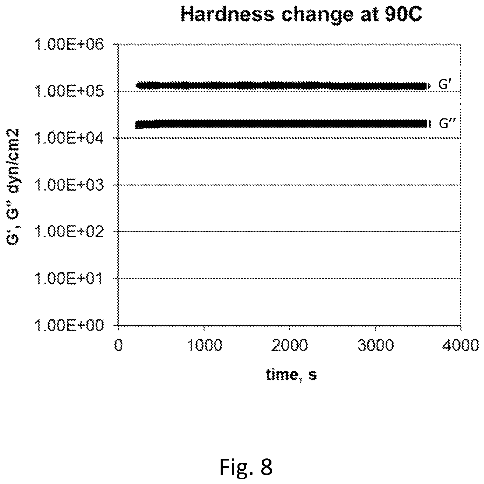

In one test, the shear storage and loss moduli of the cured polyurethane resilient material in the capstan channel were tested as a function of time at elevated temperature. The test was performed with an Ares rotational rheometer using a 25 mm parallel plate geometry. The shear rate was 1 rad/s and the shear strain was 2%. Two days after curing, the capstan was heated to 90 °C and the shear storage and loss moduli of the polyurethane resilient material were measured. A temperature of 90 °C was selected for the test because it is a representative temperature encountered by the capstan during typical fiber processing conditions. The shear storage and loss moduli are indicators of hardness. Variability in shear storage and loss moduli lead to variability in hardness. FIG. 8 shows the shear storage modulus (G') and shear loss modulus (G") of the polyurethane resilient material as a function of time at 90 °C. The data show that the shear storage and loss moduli of the polyurethane resilient material are stable over time at 90 °C.

In a continuous fiber process, the capstans are rotating and contact of the fiber with the capstan introduces a shear stress to the capstan. In the present integrated capstans, a fiber-engaging material is placed over the capstan and experiences stresses related to fiber draw and conveyance. To maintain process stability, the hardness of the polyurethane resilient material needs to remain invariant under the stress levels encountered during fiber processing and testing.

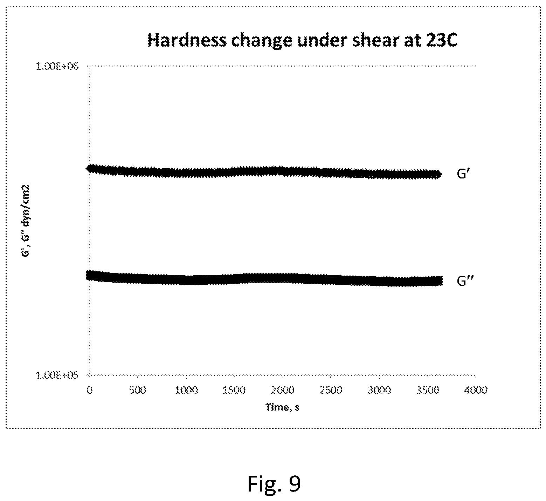

In a second test, the shear storage and loss moduli of the cured polyurethane resilient material were tested under shear rate of 10 rad/s at room temperature. The test was performed with an Ares rotational rheometer using a 25 mm parallel plate geometry. The shear strain was 5%. FIG. 9 shows the time dependence of the shear storage modulus and shear loss modulus of the polyurethane resilient material on the integrated capstan. The test was performed at room temperature two days after curing the polyurethane resilient material. The data indicate that the shear storage and loss moduli of the polyurethane resilient material are stable under shear.

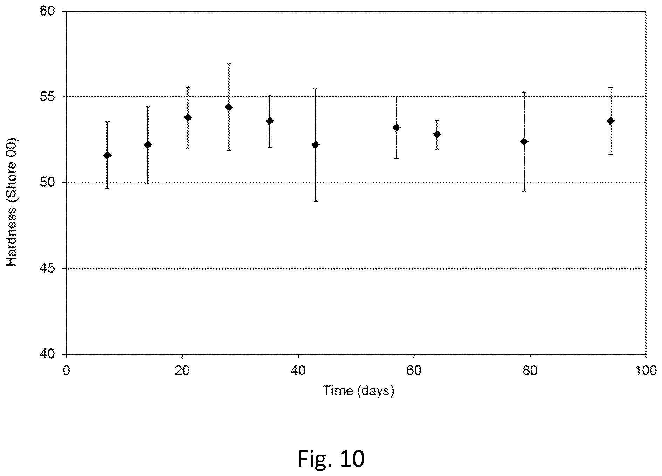

FIG. 10 shows durometer hardness measurements over time at room temperature for the polyurethane resilient material. The test was performed with a standard Shore 00 durometer. The hardness of the polyurethane resilient material was determined at eight different points around the circumference of the capstan, the average of the eight hardness measurements was taken as a data point and reported in FIG. 10. The data indicate that the hardness remains stable over a time period of several days.

Unless otherwise expressly stated, it is in no way intended that any method set forth herein be construed as requiring that its steps be performed in a specific order. Accordingly, where a method claim does not actually recite an order to be followed by its steps or it is not otherwise specifically stated in the claims or descriptions that the steps are to be limited to a specific order, it is no way intended that any particular order be inferred.

It will be apparent to those skilled in the art that various modifications and variations can be made to the embodiments described herein without departing from the scope of the claimed subject matter. Thus it is intended that the specification cover the modifications and variations of the various embodiments described herein provided such modification and variations come within the scope of the appended claims.

## Claims

1. An integrated capstan comprising:
a capstan (105. 106); and
a fiber-engaging material (120), wherein said fiber-engaging material is formed on the surface of said capstan, **characterized in that**
said fiber-engaging material having a hardness in the range from 40 Shore 00 to 70 Shore 00.

2. The integrated capstan of claim 1, wherein said capstan includes a channel (125), said fiber-engaging material occupying said channel.

3. The integrated capstan of claim 1 or 2, wherein said fiber-engaging material comprises a polyurethane material.

4. The integrated capstan of claim 3, wherein said polyurethane material comprises the cured product of a formulation that includes an isocyanate component and a hydroxy component.

5. The integrated capstan of claim 4, wherein said isocyanate component includes a di-functional isocyanate compound.

6. The integrated capstan of claim 5, wherein said di-functional isocyanate compound is a polymer containing urethane groups.

7. The integrated capstan of claim 6, wherein said polymer is a polyether.

8. The integrated capstan of claim 6 or 7, wherein said polymer is polybutadiene.

9. The integrated capstan of any of claims 3-8, wherein said fiber-engaging material further comprises a capping layer (123), said capping layer formed on said polyurethane material.

10. The integrated capstan of claim 9, wherein said capping layer has a hardness within ± 10 Shore 00 of the hardness of said polyurethane material.

11. The integrated capstan of claim 9 or 10, wherein said capping layer comprises the cured product of a formulation that includes an acrylate compound.

12. The integrated capstan of any of claims 1-8, wherein said fiber-engaging material comprises a resilient material (121) and a capping layer (123) formed on said resilient material, said resilient material having a hardness in the range from 40 Shore 00 to 70 Shore 00.

13. The integrated capstan of claim 12, wherein said capping layer (123) has a hardness within ± 10 Shore 00 of said hardness of said resilient material.

14. An apparatus (100) for processing an optical fiber comprising;
an integrated capstan (205, 206) according to claim 1; and
a fiber conveyance pathway (101), said fiber conveyance pathway positioned adjacent to said integrated capstan such that when said optical fiber is directed along said fiber conveyance pathway, said optical fiber contacts said fiber-engaging material.

15. A method for screen testing an optical fiber comprising:
drawing an optical fiber along a fiber conveyance pathway (101); and
directing said optical fiber around an integrated capstan (205, 206), said integrated capstan comprising a capstan and a fiber-engaging material (120), **characterised in that** the fiber-engaging material is formed on the surface of said capstan, said fiber-engaging material having a hardness in the range from 40 Shore 00 to 70 Shore 00, said optical fiber contacting said fiber-engaging material.

## Patentansprüche

1. Integrierter Capstan, umfassend:
einen Capstan (105, 106); und
ein Fasereingriffsmaterial (120), wobei das Fasereingriffsmaterial
auf der Oberfläche des Capstans gebildet ist, **dadurch gekennzeichnet,**
**dass** das Fasereingriffsmaterial eine Härte im Bereich
von 40 Shore 00 bis 70 Shore 00 aufweist.

2. Integrierter Capstan nach Anspruch 1, wobei der Capstan einen Kanal (125) beinhaltet, wobei das Fasereingriffsmaterial den Kanal einnimmt.

3. Integrierter Capstan nach Anspruch 1 oder 2, wobei das Fasereingriffsmaterial ein Polyurethanmaterial umfasst.

4. Integrierter Capstan nach Anspruch 3, wobei das Polyurethanmaterial das ausgehärtete Produkt einer Formulierung umfasst, die eine Isocyanatkomponente und eine Hydroxykomponente beinhaltet.

5. Integrierter Capstan nach Anspruch 4, wobei die Isocyanatkomponente eine difunktionelle Isocyanatverbindung beinhaltet.

6. Integrierter Capstan nach Anspruch 5, wobei die difunktionelle Isocyanatverbindung ein Urethangruppen enthaltendes Polymer ist.

7. Integrierter Capstan nach Anspruch 6, wobei das Polymer ein Polyether ist.

8. Integrierter Capstan nach Anspruch 6 oder 7, wobei das Polymer Polybutadien ist.

9. Integrierter Capstan nach einem der Ansprüche 3-8, wobei das Fasereingriffsmaterial ferner eine Deckschicht (123) umfasst, wobei die Deckschicht auf dem Polyurethanmaterial gebildet ist.

10. Integrierter Capstan nach Anspruch 9, wobei die Deckschicht eine Härte innerhalb von ± 10 Shore 00 der Härte des Polyurethanmaterials aufweist.

11. Integrierter Capstan nach Anspruch 9 oder 10, wobei die Deckschicht das ausgehärtete Produkt einer Formulierung umfasst, die eine Acrylatverbindung beinhaltet.

12. Integrierter Capstan nach einem der Ansprüche 1-8, wobei das Fasereingriffsmaterial ein resilientes Material (121) und eine auf dem resilienten Material gebildete Deckschicht (123) umfasst, wobei das resiliente Material eine Härte im Bereich von 40 Shore 00 bis 70 Shore 00 aufweist.

13. Integrierter Capstan nach Anspruch 12, wobei die Deckschicht (123) eine Härte innerhalb von ± 10 Shore 00 der Härte des resilienten Materials aufweist.

14. Vorrichtung (100) zum Verarbeiten einer optischen Faser, umfassend;
einen integrierten Capstan (205, 206) gemäß Anspruch 1; und
einen Fasertransportweg (101), wobei der Fasertransportweg neben dem integrierten Capstan positioniert ist, sodass, wenn die optische Faser entlang des Fasertransportwegs geführt wird, die optische Faser das Fasereingriffsmaterial berührt.

15. Verfahren für Zugspannungstest einer optischen Faser, umfassend:
Ziehen einer optischen Faser entlang eines Fasertransportwegs (101); und
Führen der optischen Faser um einen integrierten Capstan (205, 206), wobei der integrierte Capstan einen Capstan und ein Fasereingriffsmaterial (120) umfasst, **dadurch gekennzeichnet, dass** das Fasereingriffsmaterial auf der Oberfläche des Capstans gebildet ist, wobei das Fasereingriffsmaterial eine Härte im Bereich von 40 Shore 00 bis 70 Shore 00 aufweist, wobei die optische Faser das Fasereingriffsmaterial berührt.

## Revendications

1. Cabestan intégré comprenant :
un cabestan (105, 106) ; et
un matériau de contact (120) avec les fibres, ledit matériau de contact avec les fibres étant formé à la surface dudit cabestan,
**caractérisé en ce que** ledit matériau de contact avec les fibres comporte une dureté comprise entre 40 Shore 00 et 70 Shore 00.

2. Cabestan intégré selon la revendication 1, ledit cabestan comprenant un canal (125), ledit matériau de contact avec les fibres occupant ledit canal.

3. Cabestan intégré selon la revendication 1 ou 2, ledit matériau de contact avec les fibres comprenant un matériau de polyuréthane.

4. Cabestan intégré selon la revendication 3, ledit matériau de polyuréthane comprenant le produit durci d'une formulation qui comprend un composant isocyanate et un composant hydroxy.

5. Cabestan intégré selon la revendication 4, ledit composant isocyanate comprenant un composé isocyanate bifonctionnel.

6. Cabestan intégré selon la revendication 5, ledit composé isocyanate bifonctionnel étant un polymère contenant des groupes uréthane.

7. Cabestan intégré selon la revendication 6, ledit polymère étant un polyéther.

8. Cabestan intégré selon la revendication 6 ou 7, ledit polymère étant le polybutadiène.

9. Cabestan intégré selon l'une quelconque des revendications 3 à 8, ledit matériau de contact avec les fibres comprenant en outre une couche de recouvrement (123), ladite couche de recouvrement étant formée sur ledit matériau de polyuréthane.

10. Cabestan intégré selon la revendication 9, ladite couche de recouvrement comportant une dureté comprise dans les limites de ± 10 Shore 00 de la dureté dudit matériau de polyuréthane.

11. Cabestan intégré selon la revendication 9 ou 10, ladite couche de recouvrement comprenant le produit durci d'une formulation qui comprend un composé acrylate.

12. Cabestan intégré selon l'une quelconque des revendications 1 à 8, ledit matériau de contact avec les fibres comprenant un matériau résilient (121) et une couche de recouvrement (123) formée sur ledit matériau résilient, ledit matériau résilient comportant une dureté comprise entre 40 Shore 00 et 70 Shore 00.

13. Cabestan intégré selon la revendication 12, ladite couche de recouvrement (123) comportant une dureté comprise dans les limites de ± 10 Shore 00 de ladite dureté dudit matériau résilient.

14. Appareil (100) destiné à traiter une fibre optique comprenant :
un cabestan intégré (205, 206) selon la revendication 1 ; et
un trajet de transport de fibre (101), ledit trajet de transport de fibre étant positionné adjacent audit cabestan intégré de sorte que lorsque ladite fibre optique est guidée le long dudit trajet de transport de fibre, ladite fibre optique entrant en contact avec ledit matériau de contact avec les fibres.

15. Procédé permettant d'effectuer un test de sélection de fibres sur une fibre optique comprenant :
le fait de tirer une fibre optique le long d'un trajet de transport de fibre (101) ; et
le guidage de ladite fibre optique autour d'un cabestan intégré (205, 206), ledit cabestan intégré comprenant un cabestan et un matériau de contact (120) avec les fibres, **caractérisé en ce que** le matériau de contact avec les fibres est formé à la surface dudit cabestan, ledit matériau de contact avec les fibres comportant une dureté comprise entre 40 Shore 00 et 70 Shore 00, ladite fibre optique étant en contact avec ledit matériau de contact avec les fibres.
